Europäisches Patentamt

(19) European Patent Office

Office européen des brevets

(11) Publication number: **0 077 104**
**B1**

(12) **EUROPEAN PATENT SPECIFICATION**

(45) Date of publication of patent specification: **21.08.85**

(21) Application number: **82201255.5**

(22) Date of filing: **11.10.82**

(51) Int. Cl.⁴: **C 07 C 119/042,**
**C 08 G 18/02, C 08 G 18/79**

(54) **Oligomer of a diisocyanate.**

(30) Priority: **10.10.81 NL 8104623**

(43) Date of publication of application:
**20.04.83 Bulletin 83/16**

(45) Publication of the grant of the patent:
**21.08.85 Bulletin 85/34**

(84) Designated Contracting States:
**AT BE CH DE FR GB IT LI NL SE**

(56) References cited:
**US-A-3 311 654**

(73) Proprietor: **STAMICARBON B.V.**
**Postbus 10**
**NL-6160 MC Geleen (NL)**

(72) Inventor: **Van Geenen, Albert Arnold**
**Merkelbeekerstraat 82**
**NL-6441 KM Brunssum (NL)**
Inventor: **Vincent, Joseph A. J. M.**
**Hubertusstraat 73**
**NL-6191 PB Beek (NL)**

(74) Representative: **Hatzmann, Marinus Jan et al**
**OCTROOIBUREAU DSM P.O.Box 9**
**NL-6160 MA Geleen (NL)**

Courier Press, Leamington Spa, England.

## Description

The invention relates to an oligomer of an aliphatic diisocyanate.

It is known in the art that diisocyanates can be converted into oligomers thereof by reacting the diisocyanates, optionally in the presence of a catalyst. An important reason for this conversion of diisocyanates into the corresponding oligomers lies in the properties of the diisocyanate.

The fact is that diisocyanates are rather volatile and, moreover, toxic. By converting the diisocyanate into its oligomer a product is obtained which is easier to handle and, moreover, far less volatile.

One of the most frequently used aliphatic diisocyanates is 1,6 hexanediisocyanate. In the oligomerization of this compound problems are encountered.

If an effort is made to make an oligomer thereof in the usual manner, it will be found that, at a high degree of conversion, gelation of the reaction mixture will occur. It is possible, however, at a low degree of conversion for the non-reacted diisocyanate to be distilled off. Such a process is laborious, however, and very expensive energetically.

An alternative for this oligomerization is the formation of biuret. In this process a reaction product of two or more diisocyanate molecules is formed under the influence of water. A disadvantage of this process, however, is that it is difficult to reach a low free diisocyanate content. Moreover, this reaction is reversible, so that after some time the free diisocyanate content will rise.

As a consequence extra safety precautions must be taken in the processing of this product.

An object of the invention is to provide an oligomer of a diisocyanate without encountering these problems.

Another object of the invention is to provide an oligomer that can be used longer when applied in lacquers (longer potlife).

According to the invention such an oligomer is characterized in that, as diisocyanate, a compound according to formula I of the formula sheet is used, where $R_1$ represents an alkyl group with one or more C atoms, a substituted or unsubstituted phenyl group or a group according to formula II of the formula sheet, where $R_1'$ and $R_1''$ represent alkyl groups with one or more C atoms, and where $R_2$, $R_3$ and $R_4$ are the same or different and are chosen from the group consisting of:

— alkyl group with one or more C atoms
— alkoxy group with one or more C atoms
— H, or where $R_1$ and $R_2$ from a cycloaliphatic ring with 5—12 C-atoms, and $R_3$ and $R_4$ have the above meanings.

Preferably $R_1$ is an alkyl group and $R_2$, $R_3$ and $R_4$ are hydrogen atoms. More particularly a methyl group is used for $R_1$. In that case 1,5 hexanediisocyanate is obtained.

In another preferred mode of realizing the invention $R_1$ and $R_2$ jointly form a cycloaliphatic ring. This ring contains 5—12, preferably 5, 6 or 12, C-atoms, as indicated in Formula III, wherein n=3—10.

The oligomer can be prepared in a manner known per se from the corresponding diisocyanate, for instance by heating the diisocyanate in solution or as such.

Generally, however, a catalyst is used. Suitable catalysts are tertiary amines (such as tetrazoles, N,N-dialkylpiperazine, pyridines substituted with alkoxy or amino groups), alkali metal hydroxides, alkali and alkali earth metal salts of carboxylic acids (such as calcium naphthenate), metal alkoxides, quarternary ammonium hydroxides, and the like. In certain cases a co-catalyst may be desirable.

The oligomerization can be effected at room temperature, elevated or reduced temperature. The pressure is preferably about atmospheric, while it is possible to proceed with the exclusion of air, for instance in the presence of substantially pure nitrogen, in order to eliminate the adverse effects of water.

The oligomerization can be effected in a solution of an inert solvent. Suitable solvents are polar, aprotic solvents, such as organic esters or nitrile compounds. Examples thereof are butylacetate and/or acetonitrile.

The preparation of the oligomer may be effected also under such conditions that the oligomer formes precipitates during the preparation, for instance by effecting the oligomerization in mass or in a solvent suitable for that purpose.

The oligomer may be used for commercial purposes as a solution in a solvent. The solid content of the oligomer solution then preferably amounts to 5—75% (wt), more particularly 35—60% (wt).

After the desired degree of conversion and the desired NCO content have been reached, the reaction can be stopped in a manner known per se, for instance by the addition of an acid chloride, such as benzoylchloride.

In the preparation of the oligomer the diisocyanate further defined in the claims is started from. The diisocyanate can, for instance, be prepared from the corresponding diamine by phosgenation, or in another manner known in the art.

The diamine can be obtained in the various manners known in the art. An effective manner is the process described in Chem. Ber. *99* (10) 3387—9 (1966), for, among other things 1,5 diaminohexane. An alternative manner is the hydrogenation, in ammoniacal conditions, of the corresponding ketonitrile compound.

Preferably the oligomer does not contain more than 2.0% (wt) free diisocyanate, more particularly not more than 0.5% (wt).

The content of NCO groups in the oligomer is preferably between 10 and 25% (wt). The weight average molecular weight can preferably be between 500 and 5000, more particularly between 500 and 1500.

The most suitable values for NCO content and molecular weight may vary according to application. The most suitable value according to application can be determined in a simple manner by the person skilled in the art.

Surprisingly it has been found that the oligomer according to the invention has a number of very favourable properties.

An important property first manifests itself already in the preparation of the oligomer. The fact is that it has been found that the oligomer according to the invention, unlike for instance the oligomers of 1,6 hexane-diisocyanate, can be prepared in a very simple manner and with a high degree of conversion ($\geq 99\%$).

While applying a customary oligomerization process, the oligomer is obtained from the corresponding diisocyanate with a high yield and with high selectivity. Without much trouble a product can be made having a free diisocyanate content of 0.3% (wt) at most without free diisocyanate having to be removed or without gel-building.

Moreover, the oligomerization is not reversible, so that the free diisocyanate content does not increase after some time.

Other advantages of the oligomer according to the invention are in its use in, for instance, paints and lacquers. It has been found that the use of the oligomer results in a harder coat of paint having an equally good or even better resistance against external effects compared with, for instance, a coat of paint based on 1,6 hexanediisocyanate. Moreover, a paint or lacquer based on the oligomer according to the invention has a longer potlife.

The oligomer according to the invention can in principle be used for all diisocyanate applications known in the art. Of particular importance, however, are the higher grade uses of aliphatic diisocyanates, such as industrial wood lacquers, car repair lacquers, airplane lacquers, leather lacquers, textile coatings and the like.

The invention will now be elucidated by means of a few examples.

Examples
Example I
A. Diisocyanate preparation

71 g 1,5 diaminohexane was heated in 1 l o-dichlorobenzene to 110°C. During stirring, carbon dioxide was passed over at this temperature until the take-up ceased. While carbon dioxide was being supplied continuously, the temperature was lowered by 95°C and for four hours after-reaction was effected.

After the carbamate suspension thus obtained had been cooled down to −10°C, 150 g phosgene was introduced at a rate of 100 g/h. The phosgene flow was subsequently reduced to 10 g/h and the mixture was heated at about 5°C/5 min. to 45°C. The heating was subsequently continued at 5°C/10 min. to 75°C. After the phosgene rate had been increased to 30 g/h, heating was continued at 5°C/5 min. to 130°C. At this temperature after-reaction was effected for 16 hours. After removal of residual phosgene by discharge with nitrogen, cooling was effected to room temperature. The reaction mixture was subsequently filtered and the filtrate distilled at 15 mm/Hg. After removal of the o-dichlorobenzene, the 1,5 hexanediisocyanate was distilled at 123°C/15 mm Hg. The quantity obtained was 70 g 1,5 HDI.

B. Oligomer preparation

To a 50% (wt) solution of 1,5 HDI in butylacetate/acetonitrile (1/1 vol) 1% (wt) catalyst (in respect of 1,5 HDI) was added at room temperature (DABCO-TMR) (RTM of Air Products and Chemicals) consisting of a 50% (wt) solution in dipropyleneglycol of trimethyl-N-2 hydroxypropylammoniumhexanoate.

After the reaction had proceeded until a monomer content lower than 0.2% (wt) had been reached, the reaction was stopped with benzoylchloride.

The oligomer obtained had an average molecular weight of 980 and an NCO content of 19% (wt) and a free diisocyanate content of 0.15% (wt).

Example II and comparative Example I

Starting from an approx. 50% (wt) solution of 1,5 HDI oligomer in a 1:1 butylacetate/acetonitrile mixture obtained according to Example IB, a two-component lacquer based on hydroxyacrylate was made (Lacquer I).

This lacquer was compared with a lacquer of the same composition on the basic of Desmodur N (RTM of Bayer AG), consisting of a reaction product of 1,6 hexanediisocyanate (Lacquer II)

A. Potlife

At various moments after the mixing of the two lacquer components, the viscosity of the lacquers was measured (in sec. with a 'FORD 4 cup').

TABLE 1

| Time | Lacquer[*] I | Lacquer[**] II |
|---|---|---|
| 8 hrs | 18.6 | 19.0 |
| 48 hrs | 20.8 | 22.2 |
| 120 hrs | 25.2 | 39.2 |
| Gel after | 288 hrs | 144 hrs |

[*] According to the invention
[**] Not according to the invention.

The above results are indicative of a potlife of the lacquer based on the oligomer according to the invention that is twice as long as that of the known lacquer.

B. Resistance against chemicals

1. The same lacquers as used under A were applied to a sheet of glass. After different drying periods the resistance against acetone attack was tested for 1 minute.

TABLE 2

| Time | Lacquer I | Lacquer II |
|---|---|---|
| 8 hrs | 1 | 1 |
| 24 hrs | 3 | 2 |
| 120 hrs | $3\frac{1}{2}$ | 3 |

The scale applied was:

1 very bad
2 bad
3 moderate
4 good
5 perfect

The same lacquers were applied to mahogany-veneered chipboard. After 7 days the resistance against different treatments was measured.

TABLE 3

| | Lacquer I | Lacquer II |
|---|---|---|
| demineralized water 24 hrs | 5* | 5* |
| ethanol/water 16 hrs | 5⁻ | 5⁻ |
| coffee 16 hrs | 5 | 5 |
| lipstick 16 hrs | 5 | 5 |
| acetic acid (10%) 16 hrs | 5 | 5 |
| acetone 1 min | 4 | 3 |

*See table 2.

These results show that the resistance of the lacquer based on the oligomer according to the invention is at least as good as that of the lacquer based on products known in the art.

**Claims for the Contracting States BE CH DE FR GB IT LI NL SE**

1. Oligomer of an aliphatic diisocyanate, characterised in that, as diisocyanate, a compound according to formula I of the formula sheet is used, where $R_1$ represents an alkyl group with one or more C atoms, a substituted or unsubstituted phenyl group or a group according to formula II of the formula sheet, where $R_1'$ and $R_1''$ represent alkyl groups with one or more C atoms, and where $R_2$, $R_3$ and $R_4$ are the same or different and are chosen from the group consisting of:

— alkyl group with one or more C atoms

— alkoxy group with one or more C atoms
— H, or

where $R_1$ and $R_2$ form a cycloaliphatic ring with 5—12 C atoms, and $R_3$ and $R_4$ have the above meanings.

2. Oligomer according to claim 1, characterized in that $R_1$ represents an alkyl group and $R_2$, $R_3$ and $R_4$ represent a hydrogen atom.

3. Oligomer according to claim 1 or 2, characterized in that $R_1$ is a methyl group.

4. Oligomer according to claim 1, characterized in that the cycloaliphatic ring contains 5, 6 or 12 C atoms.

5. Oligomer according to any one of claims 1—4, characterized in that the free diisocyanate content is not higher than 2.0% (wt), but particularly not higher than 0.5% (wt).

6. Solution of an oligomer according to any one of claims 1—5 in an inert solvent.

7. Lacquer based on an oligomer according to any one of claims 1—5.

8. Polyurethane composition obtained while applying an oligomer according to any one of claims 1—5.

**Claims for the Contracting State AT**

1. Process for the preparation of an oligomer of an aliphatic diisocyanate, characterized in that, as diisocyanate, a compound according to formula I of the formula sheet is used, where $R_1$ represents an alkyl group with one or more C atoms, a substituted or unsubstituted phenyl group or a group according to formula II of the formula sheet, where $R_1'$ and $R_1''$ represent alkyl groups with one or more C atoms, and where $R_2$, $R_3$ and $R_4$ are the same or different and are chosen from the group consisting of:

— alkyl group with one or more C atoms
— alkoxy group with one or more C atoms
— H,

where $R_1$ and $R_2$ form a cycloaliphatic ring with 5—12 C atoms, and $R_3$ and $R_4$ have the above meanings.

2. Process according to claim 1, characterized in that $R_1$ represents an alkyl group and $R_2$, $R_3$ and $R_4$ represent a hydrogen atom.

3. Process according to claim 1 or 2, characterized in that $R_1$ is a methyl group.

4. Process according to claim 1, characterized in that the cycloaliphatic ring contains 5, 6 or 12 C atoms.

5. Process according to any one of claims 1—4, characterized in that the free diisocyanate content is not higher than 2.0% (wt), but particularly not higher than 0.5% (wt).

6. Solution of an oligomer prepared according to any one of claims 1—5 in an inert solvent.

7. Lacquer based on an oligomer prepared according to any one of claims 1—5.

8. Polyurethane composition obtained while applying an oligomer prepared according to any one of claims 1—5.

## Patentansprüche für die Vertragsstaaten BE, CH, DE, FR, GB, IT, NL, SE

1. Oligomer eines aliphatischen Diisocyanats, dadurch gekennzeichnet, daß als Diisocyanat eine Verbindung der Formel I des Formelblattes verwendet wird, worin $R_1$ eine Alkylgruppe mit einem oder mehreren C-Atomen, eine substituierte oder unsubstituierte Phenylgruppe oder eine Gruppe gemäß der Formel II des Formelblattes darstellt, worin $R_1'$ und $R_1''$ Alkylgruppen mit einem oder mehreren C-Atomen darstellen und $R_2$, $R_3$ und $R_4$ gleich oder verschieden sind und ausgewählt sind aus der Gruppe bestehend aus:

— Alkylgruppe mit einem oder mehreren C-Atomen

— Alkoxygruppe mit einem oder mehreren C-Atomen

— H

oder worin $R_1$ und $R_2$ einen cycloaliphatischen Ring mit 5—12 C-Atomen bilden und $R_3$ und $R_4$ die obigen Bedeutungen besitzen.

2. Oligomer nach Anspruch 1, dadurch gekennzeichnet, daß $R_1$ eine Alkylgruppe darstellt und $R_2$, $R_3$ und $R_4$ eine Wasserstoff darstellen.

3. Oligomer nach Anspruch 1 oder 2, dadurch gekennzeichnet, daß $R_1$ eine Methylgruppe ist.

4. Oligomer nach Anspruch 1, dadurch gekennzeichnet, daß der cycloaliphatische Ring 5,6 oder 12 C-Atome enthält.

5. Oligomer nach einem der Ansprüche 1—4, dadurch gekennzeichnet, daß der Gehalt an freiem Diisocyanat nicht höher als 2,0% (Gew.), jedoch insbesondere nicht höher als 0,5% (Gew.) ist.

6. Lösung eines Oligomers nach einem der Ansprüche 1—5 in einem inerten Lösungsmittel.

7. Lack auf der Basis eines Oligomers nach einem der Ansprüche 1—5.

8. Polymethanzusammensetzung erhalten während der Aufbringung eines Oligomers nach einem der Ansprüche 1—5.

## Patentansprüche für den Vertragsstaat AT

1. Verfahren zur Herstellung eines Oligomeres eines aliphatischen Diisocyanats, dadurch gekennzeichnet, daß als Diisocyanat eine Verbindung der Formel I des Formelblattes verwendet wird, worin $R_1$ eine Alkylgruppe mit einem oder mehreren C-Atomen, eine substituierte oder unsubstituierte Phenylgruppe oder eine Gruppe gemäß der Formel II des Formelblattes darstellt, worin $R_1'$ und $R_1''$ Alkylgruppen mit einem oder mehreren C-Atomen darstellen und $R_2$, $R_3$ und $R_4$ gleich oder verschieden sind und ausgewählt sind aus der Gruppe bestehend aus:

— Alkylgruppe mit einem oder mehreren C-Atomen

— Alkoxygruppe mit einem oder mehreren C-Atomen

— H

oder worin $R_1$ und $R_2$ einen cycloaliphatischen Ring mit 5—12 C-Atomen bilden und $R_3$ und $R_4$ die obigen Bedeutungen besitzen.

2. Verfahren nach Anspruch 1, dadurch gekennzeichnet, daß $R_1$ eine Alkylgruppe darstellt und $R_2$, $R_3$ und $R_4$ ein Wasserstoff darstellen.

3. Verfahren nach Anspruch 1 oder 2, dadurch gekennzeichnet, daß $R_1$ eine Methylgruppe ist.

4. Verfahren nach Anspruch 1, dadurch gekennzeichnet, daß der cycloaliphatische Ring 5,6 oder 12 C-Atome enthält.

5. Verfahren nach einem der Ansprüche 1—4, dadurch gekennzeichnet, daß der Gehalt an freiem Diisocyanat nicht höher als 2,0% (Gew.), jedoch insbesondere nicht höher als 0,5% (Gew.) ist.

6. Lösung eines Oligomers bereitet nach einem der Ansprüche 1—5 in einem inerten Lösungsmittel.

7. Lack auf der Basis eines Oligomers bereitet nach einem der Ansprüche 1—5.

8. Polymethanzusammensetzung erhalten während der Aufbringung eines Oligomers bereitet nach einem der Ansprüche 1—5.

## Revendications pour les Etats Contractants BE, CH, DE, FR, GB, IT, NL, SE

1. Oligomère d'un diisocyanate, caractérisé en ce qu'en qualité de diisocyanate, on utilise un composé répondant à la formule I de la feuille de formules, dans laquelle $R_1$ représente un radical alkyle contenant un ou plusieurs atomes de C, un radical phényle substitué ou non substitué ou un groupe de formule II de la feuille de formules, dans laquelle $R_1'$ et $R_1''$ représentent des radicaux alkyle contenant un ou plusieurs atomes de C, et dans laquelle $R_2$, $R_3$ et $R_4$, qui sont identiques ou différents, sont choisis dans le groupe comprenant:

— un radical alkyle contenant un ou plusieurs atomes de C

— un radical alcoxy contenant un ou plusieurs atomes de C

— H, ou

$R_1$ et $R_2$ forment un noyau cycloaliphatique de 5 à 12 atomes de C, $R_3$ et $R_4$ ont les significations ci-dessus.

2. Oligomère selon la revendication 1, caractérisé en ce que $R_1$ représente un radical alkyle et $R_2$, $R_3$ et $R_4$ représentent chacun un atome d'hydrogène.

3. Oligomère selon la revendication 1 ou 2, caractérisé en ce que $R_1$ est un radical méthyle.

4. Oligomère selon la revendication 1, caractérisé en ce que le noyau cycloaliphatique contient 5, 6 ou 12 atomes de C.

5. Oligomère selon l'une quelconque des revendications 1 à 4, caractérisé en ce que la teneur en diisocyanate libre n'est pas plus élevée que 2,0% (pds), et, particulièrement, ne dépasse pas 0,5% (pds).

6. Solution d'un oligomère selon l'une quelconque des revendications 1 à 5, dans un solvant inerte.

7. Vernis formé à partir d'un oligomère selon l'une quelconque des revendications 1 à 5.

8. Composition de polyuréthane obtenue en appliquant un oligomère selon l'une quelconque des revendications 1 à 5.

**Revendications pour l'Etat Contractant AT**

1. Procédé de préparation d'un oligomère d'un diisocyanate, caractérisé en ce qu'en qualité de diisocyanate, on utilise un composé répondant à la formule I de la feuille de formules, dans laquelle $R_1$ représente un radical alkyle contenant un ou plusieurs atomes de C, un radical phényle substitué ou non substitué ou un groupe de formule II de la feuille de formules, dans laquelle $R_1'$ et $R_1''$ représentent des radicaux alkyle contenant un ou plusieurs atomes de C, et dans laquelle $R_2$, $R_3$ et $R_4$, qui sont identiques ou différents, sont choisis dans le groupe comprenant:

— un radical alkyle contenant un ou plusieurs atomes de C
— un radical alcoxy contenant un ou plusieurs atomes de C
— H, ou

$R_1$ et $R_2$ forment un noyau cycloaliphatique de 5 à 12 atomes de C, $R_3$ et $R_4$ ont les significations ci-dessus.

2. Procédé selon la revendication 1, caractérisé en ce que $R_1$ représente un radical alkyle et $R_2$, $R_3$ et $R_4$ représentent chacun un atome d'hydrogène.

3. Procédé selon la revendication 1 ou 2, caractérisé en ce que $R_1$ est un radical méthyle.

4. Procédé selon la revendication 1, caractérisé en ce que le noyau cycloaliphatique contient 5, 6 ou 12 atomes de C.

5. Procédé selon l'une quelconque des revendications 1 à 4, caractérisé en ce que la teneur en diisocyanate libre n'est pas plus élevée que 2,0% (pds), et, particulièrement, ne dépasse pas 0,5% (pds).

6. Solution d'un oligomère préparé selon l'une quelconque des revendications 1 à 5, dans un solvant inerte.

7. Vernis formé à partir d'un oligomère préparé selon l'une quelconque des revendications 1 à 5.

8. Composition de polyuréthane obtenue en appliquant un oligomère préparé selon l'une quelconque des revendications 1 à 5.

$$
\begin{array}{c}
O \\
\parallel \\
C \\
\parallel \\
N \quad\quad R_3 \quad\quad\quad\quad R_4 \\
| \quad\quad | \quad\quad\quad\quad | \\
R_1 - C - C - CH_2 - CH - CH_2 - N = C = O \\
| \quad\quad | \\
H \quad\quad R_2
\end{array}
$$

**Formula 1**

$$R_1{}^{"} - O - R_1{}' -$$

**Formula 2**

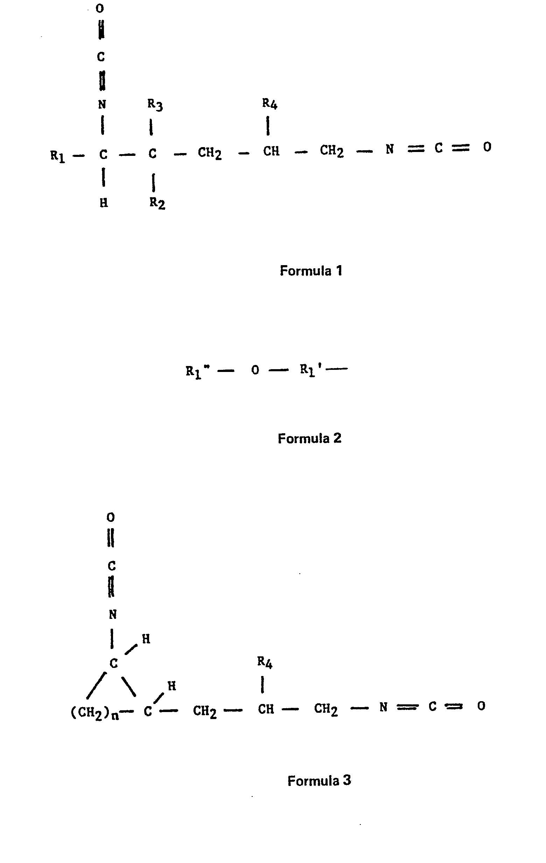

$$
\begin{array}{c}
O \\
\parallel \\
C \\
\parallel \\
N \quad H \\
| \diagup \\
C \\
\diagup \;\; \diagdown \quad H \quad\quad\quad R_4 \\
(CH_2)_n - C - CH_2 - CH - CH_2 - N = C = O
\end{array}
$$

**Formula 3**

1